# EUROPEAN PATENT APPLICATION

(11) **EP 3 062 121 A1**
(43) Date of publication of application: **31.08.2016**
(21) Application number: 16156056.0
(22) Date of filing: 17.02.2016
(51) Int. Cl.: G01R 33/28, G01R 33/54

(54) **MAGNETIC RESONANCE IMAGING (MRI) APPARATUS AND METHOD OF CONTROLLING MRI APPARATUS**

(30) Priority: 17.02.2015 KR 20150024310; 17.12.2015 KR 20150181099
(71) Applicant: Samsung Electronics Co., Ltd., Gyeonggi-do 16677 (KR)
(72) Inventor: KIM, Joon-soo, Seoul 209-602 (KR); JO, Jae-moon, Gyeonggi-do C-2803 (KR)
(74) Representative: Jacobs, Bart

(57) **Abstract**

A magnetic resonance imaging (MRI) apparatus and a method of controlling the MRI apparatus are provided. The MRI apparatus includes a signal transceiver configured to transmit a radio frequency (RF) signal to an object, and receive a magnetic resonance (MR) signal from the object. The MRI apparatus further includes a controller configured to control the signal transceiver based on sequence information that is used according to an imaging protocol, determine a degree at which the object is able to move based on the sequence information, to generate operation information, and control a device connected to the MRI apparatus to provide the operation information. In one embodiment, noting that object motion during the acquisition of central k-space data results in higher motion artefact power than object motion during the acquisition of peripheral k-space data, the invention proposes to display an image which induces a concentration of the object during the acquisition of the central k-space data, thereby reducing the degree of object motion during the acquisition of the central k-space data.

## Description

### CROSS-REFERENCE TO RELATED APPLICATIONS

This application claims priority from Korean Patent Application No. 10-2015-0024310, filed on February 17, 2015, and Korean Patent Application No. 10-2015-0181099, filed on December 17, 2015, in the Korean Intellectual Property Office, the disclosures of which are incorporated herein by reference in their entireties.

### BACKGROUND

### 1. Field

Apparatuses and methods consistent with exemplary embodiments relate to magnetic resonance imaging (MRI) apparatuses and methods of controlling an MRI apparatus.

### 2. Description of the Related Art

An MRI apparatus uses a magnetic field to capture an image of a target object, and is widely used in the accurate diagnosis of diseases because it shows stereoscopic images of bones, lumbar discs, joints, nerve ligaments, etc. at angles.

The MRI apparatus is configured to acquire MR signals and reconstruct the acquired MR signals into an image to be output. The MRI apparatus uses different pulse sequences according to MR images to be obtained.

To obtain an MR image, a target object is used to enter a confined space and undergo scanning for more than several minutes. However, it may be difficult for the target object to remain stationary for a few minutes during the scanning process. Movement of the target object during the scanning process may make it difficult to obtain high quality images.

### SUMMARY

Exemplary embodiments may address at least the above problems and/or disadvantages and other disadvantages not described above. Also, the exemplary embodiments are not required to overcome the disadvantages described above, and may not overcome any of the problems described above.

One or more exemplary embodiments provide magnetic resonance imaging (MRI) apparatuses and methods of controlling an MRI apparatus, whereby high quality MR images may be obtained by minimizing motion of a target object during scanning.

According to an aspect of an exemplary embodiment, there is provided an MRI apparatus including a signal transceiver configured to transmit a radio frequency (RF) signal to an object, and receive a magnetic resonance (MR) signal from the object. The MRI apparatus further includes a controller configured to control the signal transceiver based on sequence information that is used according to an imaging protocol, determine a degree at which the object is able to move based on the sequence information, to generate operation information, and control a device connected to the MRI apparatus to provide the operation information.

The controller may be further configured to determine information of whether the object is able to move based on a time interval.

The device may include a display configured to display the operation information to the object as an image.

The controller may be further configured to control the display to display the operation information to the object during a time interval when the object is able to move.

The controller may be further configured to control the display to display an image for inducing a concentration of the object while the signal transceiver is receiving MR signals of a low frequency region in a k-space, and display an image for helping the object relax while the signal transceiver is receiving MR signals of a high frequency region in the k-space.

The controller may be further configured to control the display to display an image showing a level of progress of an examination of the object based on the operation information.

The image may include at least one among a progress bar and a text message.

The image may distinguish a first time interval when the object is to remain stationary from a second time interval when the object is able to move.

The MRI apparatus may further include a bore in which the object is disposed, and the display may include a first display disposed in the bore, a second display disposed outside the bore, a first projector disposed in the bore, and a second projector disposed outside the bore.

The MRI apparatus may further include a bore in which the object is disposed, and an immobilizer disposed in the bore, and configured to immobilize motion of the object. The controller may be further configured to control the immobilizer based on the sequence information.

The controller may be further configured to control the immobilizer to immobilize a part of the object to be measured based on the sequence information.

The controller may be further configured to control the immobilizer to immobilize a part of the object to be measured during a time interval when the object is to remain stationary.

The sequence information may include a strength and an application timing of a pulse signal for generating a gradient magnetic field around the object.

According to an aspect of another exemplary embodiment, there is provided a method of controlling a magnetic resonance imaging (MRI) apparatus, the method including transmitting a radio frequency (RF) signal to an object, and receiving a magnetic resonance (MR) signal from the object, based on sequence information that is used according to an imaging protocol. The method further includes determining a degree at which the object is able to move based on the sequence information, to generate operation information, and providing the operation information.

The determining may include determining information of whether the object is able to move based on a time interval.

The providing may include displaying the operation information to the object as an image.

The providing may include displaying the operation information to the object during a time interval when the object is able to move.

The providing may include displaying an image for inducing a concentration of the object while the signal transceiver is receiving MR signals of a low frequency region in a k-space, and displaying an image for helping the object relax while the signal transceiver is receiving MR signals of a high frequency region in the k-space.

The providing may include displaying an image showing a level of progress of an examination of the object based on the operation information.

The image may include at least one among a progress bar and a text message.

The image may distinguish a first time interval when the object is to remain stationary from a second time interval when the object is able to move.

The method may further include immobilizing motion of the object based on the sequence information.

The immobilizing may include immobilizing a part of the object to be measured based on the sequence information.

The immobilizing may include immobilizing a part of the object to be measured during a time interval when the object is to remain stationary.

The sequence information may include a strength and an application timing of a pulse signal for generating a gradient magnetic field around the object.

According to an aspect of another exemplary embodiment, there is provided a magnetic resonance imaging (MRI) apparatus including a signal transceiver configured to transmit a radio frequency (RF) signal to an object, and receive a magnetic resonance (MR) signal from the object. The MRI apparatus further includes a controller configured to control the signal transceiver based on sequence information that is used according to an imaging protocol, and determine a degree at which the object is able to move based on the sequence information, to generate the operation information. The MRI apparatus further includes a display configured to display an image to the object based on the operation information.

The controller may be further configured to control the display to display an image for inducing a concentration of the object based on the operation information.

The controller may be further configured to control the display to display an image showing a level of progress of an examination of the object based on the operation information.

According to an aspect of another exemplary embodiment, there is provided a method of controlling a magnetic resonance imaging (MRI) apparatus, the method including transmitting a radio frequency (RF) signal to an object, and receiving a magnetic resonance (MR) signal from the object, based on sequence information that is used according to an imaging protocol, determining a degree at which the object is able to move based on the sequence information, to generate the operation information, and displaying an image to the object based on the operation information.

The displaying may include displaying an image for inducing a concentration of the object based on the operation information.

The displaying may include displaying an image showing a level of progress of an examination of the object based on the operation information.

According to an aspect of another exemplary embodiment, there is provided a magnetic resonance imaging (MRI) apparatus including a signal transceiver, and a controller configured to control the signal transceiver to transmit a radio frequency (RF) signal to an object, and receive a magnetic resonance (MR) signal from the object, based on a sequence. The controller is further configured to determine whether the object is able to move based on the sequence, and control a device connected to the MRI apparatus based on the determination.

The device may include a display, and the controller may be further configured to control the display to display an image for inducing a concentration of the object in response to the controller determining that the object is not to move, and display an image for helping the object relax in response to the controller determining that object is able to move.

The device may include a display, and the controller may be further configured to control the display to display a bar showing a level of progress of an examination of the object, display a text indicating that the object is not to move in response to the controller determining that the object is not to move, and display a text indicating the object is able to move in response to the controller determining that the object is able to move.

The device may include an immobilizer, and the controller may be further configured to control the immobilizer to immobilize a part of the object to be measured in response to the controller determining that the object is not to move.

### BRIEF DESCRIPTION OF THE DRAWINGS

The above and/or other aspects will be more apparent by describing exemplary embodiments with reference to the accompanying drawings, in which:
FIG. 1 is a block diagram of a magnetic resonance imaging (MRI) apparatus according to an exemplary embodiment;
FIG. 2 is a flowchart of a method of controlling an MRI apparatus according to an exemplary embodiment;
FIG. 3 is a block diagram of an MRI apparatus according to another exemplary embodiment;
FIG. 4 is a flowchart of a method of controlling an MRI apparatus according to another exemplary embodiment;
FIGS. 5A and 5B are diagrams for explaining a method of controlling an MRI apparatus according to an exemplary embodiment;
FIGS. 6A, 6B, 6C, 6D, and 6E are images provided to a target object via a display of an MRI apparatus, according to an exemplary embodiment;
FIG. 7 is a flowchart of a method of controlling an MRI apparatus according to another exemplary embodiment;
FIGS. 8A and 8B are images provided to a target object via a display of an MRI apparatus, according to another exemplary embodiment;
FIG. 9 is a block diagram of an MRI apparatus according to another exemplary embodiment;
FIG. 10 is a flowchart of a method of controlling an MRI apparatus according to another exemplary embodiment;
FIGS. 11A and 11B are perspective views of immobilizers in an MRI apparatus according to exemplary embodiments;
FIG. 12 is a schematic diagram of an MRI system; and
FIG. 13 illustrates a configuration of a communication unit according to an exemplary embodiment.

### DETAILED DESCRIPTION

Exemplary embodiments are described in greater detail below with reference to the accompanying drawings.

In the following description, like drawing reference numerals are used for like elements, even in different drawings. The matters defined in the description, such as detailed construction and elements, are provided to assist in a comprehensive understanding of the exemplary embodiments. However, it is apparent that the exemplary embodiments can be practiced without those specifically defined matters. Also, well-known functions or constructions may not be described in detail because they would obscure the description with unnecessary detail.

It will be understood that the terms "comprises" and/or "comprising" used herein specify the presence of stated features or components, but do not preclude the presence or addition of one or more other features or components. In addition, the terms such as "unit," "-er (-or)," and "module" described in the specification refer to an element for performing at least one function or operation, and may be implemented in hardware, software, or the combination of hardware and software.

In the present specification, an "image" may refer to multi-dimensional data composed of discrete image elements (e.g., pixels in a two-dimensional (2D) image and voxels in a three-dimensional (3D) image). For example, the image may be a medical image of an object captured by an X-ray apparatus, a computed tomography (CT) apparatus, a magnetic resonance imaging (MRI) apparatus, an ultrasound diagnosis apparatus, or another medical imaging apparatus.

Furthermore, in the present specification, an "object" may be a human, an animal, or a part of a human or animal. For example, the object may be an organ (e.g., the liver, the heart, the womb, the brain, a breast, or the abdomen), a blood vessel, or a combination thereof. Furthermore, the "object" may be a phantom. The phantom means a material having a density, an effective atomic number, and a volume that are approximately the same as those of an organism. For example, the phantom may be a spherical phantom having properties similar to the human body.

Furthermore, in the present specification, a "user" may be, but is not limited to, a medical expert, such as a medical doctor, a nurse, a medical laboratory technologist, or a technician who repairs a medical apparatus.

Furthermore, in the present specification, an "MR image" refers to an image of an object obtained by using the nuclear magnetic resonance principle.

Furthermore, in the present specification, a "pulse sequence" refers to continuity of signals repeatedly applied by an MRI apparatus. The pulse sequence may include a time parameter of a radio frequency (RF) pulse, for example, repetition time (TR) or echo time (TE).

Furthermore, in the present specification, a "pulse sequence schematic diagram" shows an order of events that occur in an MRI apparatus. For example, the pulse sequence schematic diagram may be a diagram showing an RF pulse, a gradient magnetic field, an MR signal, or the like according to time.

An MRI system is an apparatus for acquiring a sectional image of a part of an object by expressing, in a contrast comparison, a strength of a MR signal with respect to an RF signal generated in a magnetic field having a strength. For example, if an RF signal that only resonates an atomic nucleus (for example, a hydrogen atomic nucleus) is emitted for an instant toward the object placed in a strong magnetic field and then such emission stops, an MR signal is emitted from the atomic nucleus, and thus the MRI system may receive the MR signal and acquire an MR image. The MR signal denotes an RF signal emitted from the object. An intensity of the MR signal may be determined according to a density of a predetermined atom (for example, hydrogen) of the object, a relaxation time T1, a relaxation time T2, and a flow of blood or the like.

MRI systems include characteristics different from those of other imaging apparatuses. Unlike imaging apparatuses such as CT apparatuses that acquire images according to a direction of detection hardware, MRI systems may acquire 2D images or 3D volume images that are oriented toward an optional point. MRI systems do not expose objects or examiners to radiation, unlike CT apparatuses, X-ray apparatuses, position emission tomography (PET) apparatuses, and single photon emission CT (SPECT) apparatuses, may acquire images having high soft tissue contrast, and may acquire neurological images, intravascular images, musculoskeletal images, and oncologic images that are used to precisely capturing abnormal tissues.

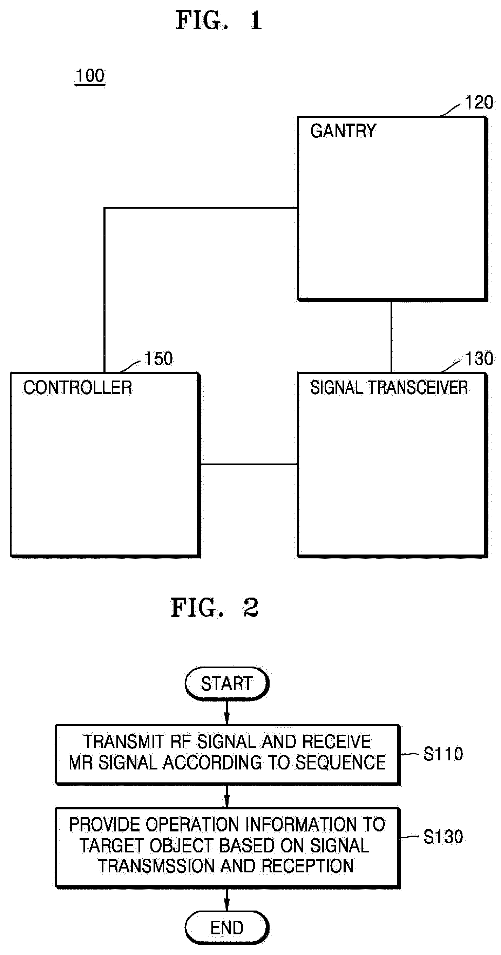

FIG. 1 is a block diagram of an MRI apparatus 100 according to an exemplary embodiment.

Referring to FIG. 1, the MRI apparatus 100 according to an exemplary embodiment includes a gantry 120, a signal transceiver 130, and a controller 150.

The gantry 120 blocks electromagnetic waves generated by a main magnet, a gradient coil, and an RF coil from being externally emitted. A magnetostatic field and a gradient magnetic field are formed at a bore in the gantry 120, and an RF signal is irradiated toward a target object.

The gantry 120 may further include displays respectively mounted outside and inside the gantry 120. The gantry 120 may provide predetermined information to the user or the target object via the displays respectively disposed outside and inside the gantry 120. For example, the display disposed outside the gantry 120 may be configured to provide an image to the user via a mirror attached to a head coil.

The gantry 120 may further include in-bore and out-bore projectors respectively located inside and outside the bore. For example, the in-bore projector may be designed to shield against magnetic fields. The out-bore projector may project an image onto an inner wall of the bore, e.g., via a mirror disposed on a table.

The controller 150 may control the gantry 120 and devices installed on the gantry 120. For example, the controller 150 may control displays mounted to the gantry 120.

In detail, the controller 150 may control displays respectively disposed outside and inside the gantry 120. The controller 150 may control an on/off status of the displays, screens to be output to the displays, etc., via a display controller.

The controller 150 may control an RF signal transceiver according to a predetermined sequence. In detail, the controller 150 may control the signal transceiver 130 according to a predetermined sequence. In this case, the pulse sequence may include all pieces of information for controlling a gradient amplifier, an RF transmitter, an RF receiver, and a transmission/reception switch, such as a strength, an application time, and application timing of a pulse signal applied to a gradient coil.

The signal transceiver 130 may transmit an RF signal and receive an MR signal. The signal transceiver 130 may control a gradient magnetic field formed inside the gantry 120, i.e., in the bore, according to a predetermined MR sequence, and control transmission and reception of an RF signal and an MR signal.

The signal transceiver 130 may be controlled by the controller 150 to transmit an RF signal to and receive an MR signal from a target object within the gantry 120. Information about control operation may be input to the controller 150, and the controller 150 may receive the information to control devices (e.g., displays and other mechanical devices) installed on the gantry 120.

According to one or more exemplary embodiments, the controller 150 may include a plurality of components having different functions. For example, the controller 150 may include a gantry controller for controlling the gantry 120 and a sequence controller for controlling the signal transceiver 130.

According to an exemplary embodiment, the controller 150 may control devices mounted on the gantry 120 to provide operation information to the target object according to sequence information.

In the present specification, operation information may include information for distinguishing a time interval during which motion of a target object may significantly affect the quality of an MR image from a time interval during which motion of the target object may not significantly affect the quality of the MR image. The operation information may further include not only visual information such as images and auditory information such as audio but also an operation of a mechanical device. For example, the operation information may include information about movement of an immobilizer as described below.

For example, the controller 150 may control displays or other various devices mounted on a table. Furthermore, the displays or other various devices mounted on the table may supply operation information to the target object or help prevent movement of the target object.

Furthermore, the displays or the other various devices may provide a time interval during which the target object is able to move. In the present specification, the time interval when the target is able to move may mean a time interval when motion of the target object does not greatly affect MR image quality. For example, the time interval during which the target object is able to move may be a time interval during which data corresponding to high frequencies in a k-space is acquired.

FIG. 2 is a flowchart of a method of controlling the MRI apparatus 100 according to an exemplary embodiment.

Referring to FIG. 2, in operation S110, the MRI apparatus 100 transmits an RF signal to and receives an MR signal from a target object according to a sequence.

In operation S130, the MRI apparatus 100 provides operation information to the target object based on the signal transmission and reception. For example, the MRI apparatus 100 may provide operation information to the target object based on a pulse sequence. In detail, a display of the MRI apparatus 100 may provide the target object with an image based on the operation information.

For example, the MRI apparatus 100 may provide operation information via a displayed image during a time interval when an object (e.g., the target object) has to remain stationary. The operation information may be provided simultaneously to an operator and the target object.

Thus, the target object may receive the operation information and make a conscious effort not to move, and the MRI apparatus 100 may obtain a high quality image by scanning the motionless object.

For example, while the signal transceiver 130 is receiving MR signals corresponding to a low frequency region in a k-space, a display may provide an image that may induce a concentration of the target object. Various images may be used to induce a concentration of the target object. Furthermore, the image may be selected based on user modeling information of the target object. In this case, the target object may remain motionless by concentrating on the image, and the MRI apparatus 100 may obtain a high quality image by scanning a motionless object.

Furthermore, the display may provide the target object with an image showing a level of progress of an examination based on an operation of the controller 150. For example, the display may provide an operation of a pulse sequence via at least one of a progress bar and text information. As another example, the display may display a progress bar in such a manner as to distinguish a time interval when the target object has to remain stationary from another time interval. Thus, the target object may make a conscious effort not to move based on an image showing a level of progress of an examination (e.g., a progress bar), and the MRI apparatus 100 may obtain a high quality image by scanning a motionless object.

Furthermore, the gantry 120 may include therein an immobilizer configured to prevent movement of the target object based on signal transmission and reception. For example, the immobilizer may immobilize a part of the target object being measured, based on signal transmission and reception.

For example, if the MRI apparatus 100 captures an image of a target object's head, the immobilizer may immobilize the head based on signal transmission and reception. If the MRI apparatus 100 captures an image of the target object's heart, the immobilizer may immobilize a target object's chest based on signal transmission and reception.

Thus, the MRI apparatus 100 may provide operation information to the target object based on signal transmission and reception, so that the target object may recognize an imaging situation and make a conscious effort to minimize movement, and the MRI apparatus 100 may obtain a high quality image by scanning a motionless target object.

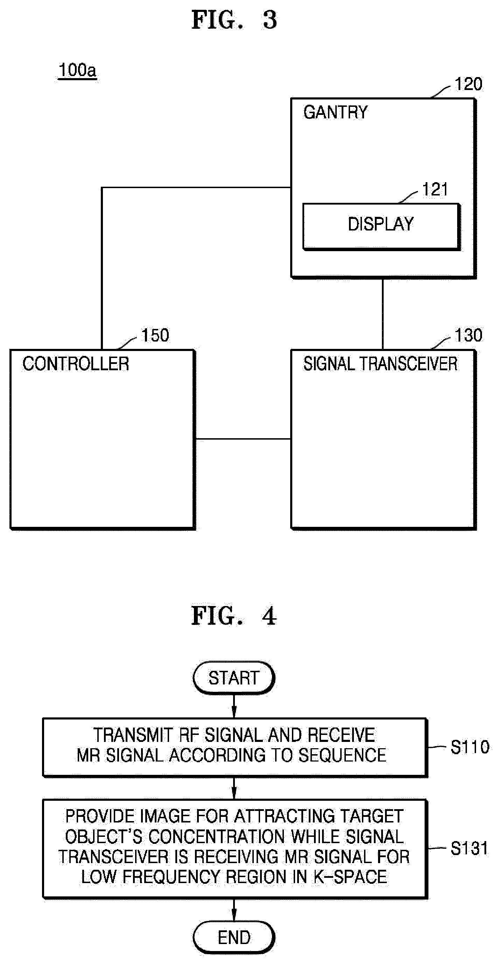

FIG. 3 is a block diagram of an MRI apparatus 100a according to another exemplary embodiment.

Referring to FIG. 3, the MRI apparatus 100a according to another exemplary embodiment includes gantry 120, the signal transceiver 130, and the controller 150. Furthermore, the gantry 120 includes a display 121.

The controller 150 and the signal transceiver 130 shown in FIG. 3 may operate in a similar way to their counterparts described with reference to FIG. 1.

The signal transceiver 130 may be controlled by the controller 150 to transmit an RF signal to and receive an MR signal from a target object within the gantry 120. Information about control operation may be input to the controller 150, and the controller 150 may receive the information to control the display 121.

The display 121 may include an in-bore display, an out-bore display, an in-bore projector, and an out-bore projector. For example, the out-bore display may be installed to provide a user with an image via a mirror attached to a head coil. For example, the in-bore projector may operate in a manner that shields against magnetic fields. The out-bore projector may project an image onto an inner wall of the bore, e.g., via a mirror disposed on a table.

The gantry 120 may further include displays disposed outside and inside the gantry 120. The MRI apparatus 100a may provide predetermined information to the user or target object via the displays disposed outside and inside the gantry 120.

The controller 150 may control the gantry 120 and the display 121 installed in the gantry 120. The controller 150 may control the on/off status of the displays, screens to be output to the displays, etc., via a display controller.

An operation of the display 121 will now be described in detail with reference to FIGS. 4 through 8B.

FIG. 4 is a flowchart for explaining a method of controlling the MRI apparatus 100a according to another exemplary embodiment.

Referring to FIG. 4, in operation S110, the MRI apparatus 100a transmits an RF signal to and receives an MR signal from a target object according to a sequence.

In operation S131, the MRI apparatus 100a provides operation information to the target object based on the signal transmission and reception. For example, while the signal transceiver 130 is receiving an MR signal for a low frequency region in a k-space, the display 121 provides an image for inducing a concentration or an attention of the target object. According to another exemplary embodiment, the display 121 may provide an image simultaneously to an operator or target object.

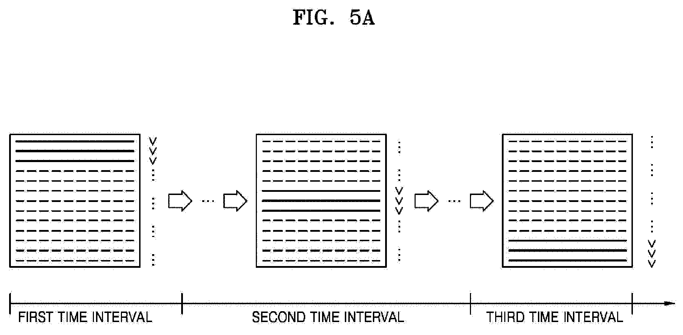

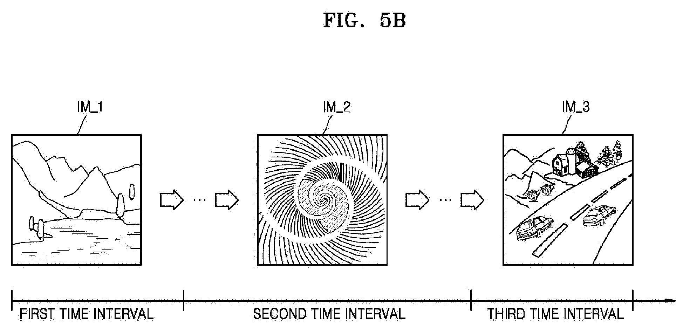

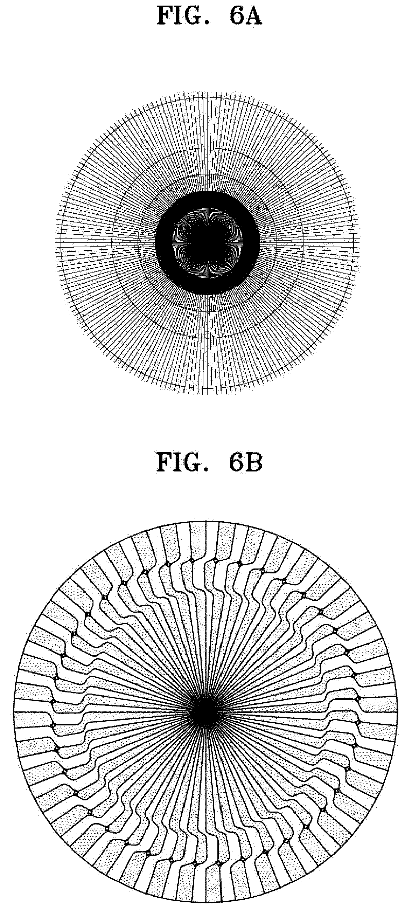

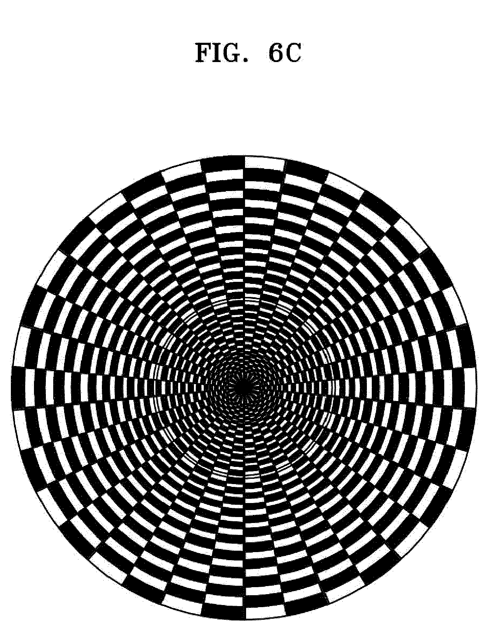

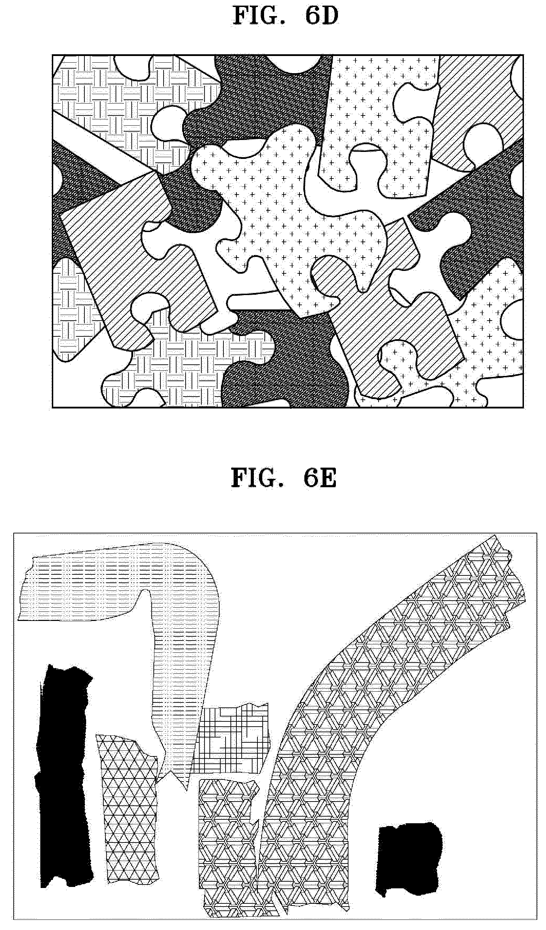

FIGS. 5A and 5B are diagrams for explaining a method of controlling the MRI apparatus 100a according to an exemplary embodiment. FIGS. 6A, 6B, 6C, 6D, and 6E are images provided to a target object via the display 121 of the MRI apparatus 100a, according to an exemplary embodiment.

Referring to FIGS. 3 and 5A, the controller 150 may receive MR signals corresponding to different frequency regions according to time intervals via the signal transceiver 130. For example, as shown in FIG. 5A, the controller 150 may receive an MR signal for a high frequency region during a first time interval (e.g., an interval corresponding to a lapse of about 30 seconds after the start of a scan), an MR signal corresponding to a low frequency region during a second time interval (e.g., an interval corresponding to a lapse of about 1 minute after the start of the scan), and, again, an MR signal corresponding to the high frequency region during a third time interval (e. g., an interval corresponding to a lapse of about 30 minutes after the start of the scan).

In addition, because the quality of an MR image is influenced by a signal for a low frequency region more than by a signal for a high frequency region, motion of a target object during the second time interval may significantly degrade the quality of an MR image.

Thus, as shown in FIG. 5B, the display 121 may provide a first image IM_1 that may help the target object relax during the first time interval, a second image IM_2 that induces a concentration of the target object during the second time interval, and a third image IM_3 that may help the target object relax during the third time interval.

However, images provided by the display 121 are not limited to the first through third images IM_1 through IM_3 shown in FIG. 5B and include other various images. For example, various types of images shown in FIGS. 6A through 6E may be used to attract the target object's concentration.

Furthermore, images provided by the display 121 may be determined based on information about the target object. For example, the first through third images IM_1 through IM_3 may be determined based on the age, gender, occupation, etc., of the target object. Furthermore, the display 121 may display an image for inducing a concentration of the target object regardless of information about the target object.

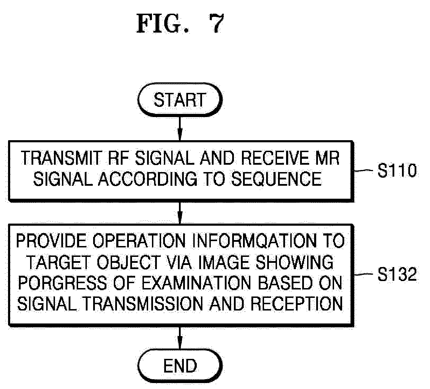

FIG. 7 is a flowchart of a method of controlling the MRI apparatus 100a according to another exemplary embodiment.

Referring to FIG. 7, in operation S110, the MRI apparatus 100a transmits an RF signal to and receives an MR signal from a target object according to a sequence.

In operation S132, the display 121 provides operation information to the target object via an image (e.g., a progress bar or text message) showing a level of progress of an examination based on the signal transmission and reception.

For example, the display 121 may display a progress bar by which the target object or an operator may recognize the operation information during or a few seconds before a time interval when the target object is able to move.

Furthermore, the display 121 may provide operation information to the target object by using text together with a progress bar.

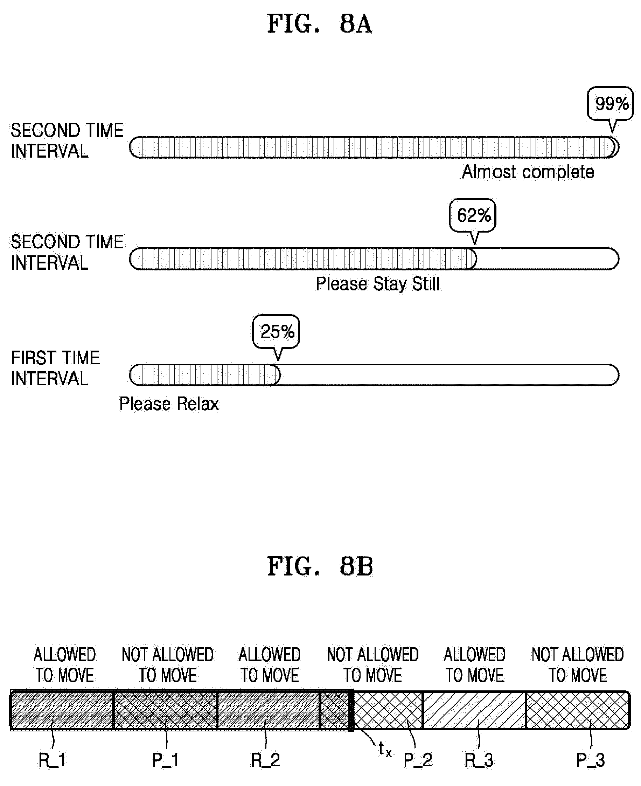

FIGS. 8A and 8B are images provided to a target object via the display 121 of the MRI apparatus 100a, according to another exemplary embodiment.

Referring to FIG. 8A, the display 210 may display a progress bar together with text <Please Relax> during a first time interval, display a progress bar together with text <Please Stay Still> during a second time interval, and display a progress bar together with text <Almost complete> during a third time interval.

According to an exemplary embodiment, a shape, a form, and a color of the progress bar as well as content, a font, and a size of text do not limit the scope of the exemplary embodiments. Furthermore, the time intervals may be classified in various ways.

Furthermore, referring to FIG. 8B, the MRI apparatus 100a may transmit an RF signal based on a sequence used according to an imaging protocol and receive an MR signal from a target object. The display 121 may provide information about whether the target object is able to move by using a progress bar. In detail, the progress bar may indicate information about first through third protocol photographing intervals P_1 through P_3. Furthermore, the progress bar may indicate information about first through third rest intervals R_1 through R_3, each being interposed between consecutive protocol photographing intervals. The progress bar may provide the target object with information about a current level tₓ of progress of examination.

By using the progress bar, the display 120 may provide to the target object, in the form of text, information indicating that the target object is not allowed to move during the first through third photographing intervals P_1 through P_3 and information indicating that the target object is allowed to move during the rest intervals R_1 through R_3. For example, the progress bar may indicate information in the form of text "Not Allowed to Move" during the first through third protocol photographing intervals P_1 through P_3 and indicate information in the form of text "Allowed to Move" during the rest intervals R_1 through R_3. However, the method of providing information via the progress bar is not limited thereto, and the progress bar may indicate the information in other forms such as by using voice or sound.

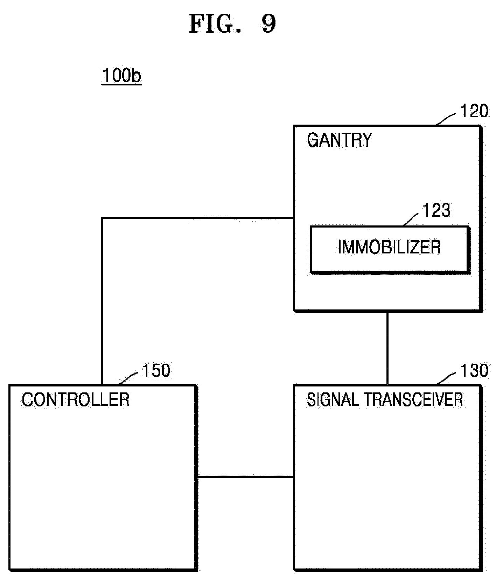

FIG. 9 is a block diagram of an MRI apparatus 100b according to another exemplary embodiment.

Referring to FIG. 9, the MRI apparatus 100b according to another exemplary embodiment includes the gantry 120, the signal transceiver 130, and the controller 150. Furthermore, the gantry 120 includes an immobilizer 123.

The controller 150 and the signal transceiver 130 may operate in a similar way to their counterparts described with reference to FIG. 1.

The gantry 120 may immobilize a target object via the immobilizer 123 according to a time interval. Thus, the MRI apparatus 100b may obtain a high quality image by immobilizing the target object directly during a time interval. In other words, the MRI apparatus 100b may minimize motion of the target object by preventing movement of the target object via the immobilizer 123 during a time interval that is highly related to image quality.

The controller 150 may control the gantry 120 and the immobilizer 123 mounted to the gantry 120. In detail, the controller 150 may control an operation of the immobilizer 123 via a table controller.

For example, the controller 150 may control the immobilizer 123 to prevent movement of a target object's head during a first time interval so that motion of the target object's head is minimized. Furthermore, the controller 150 may control the immobilizer 123 to prevent movement of a target object's torso during a second time interval so that motion of the target object's torso is minimized.

According to an exemplary embodiment, the controller 150 may control the immobilizer 123 to immobilize the target object according to sequence information.

The signal transceiver 130 may be controlled by the controller 150 to transmit an RF signal to and receive an MR signal from the target object within the gantry 120. Information about control operation may be input to the controller 150, and the controller 150 may receive the information to control the immobilizer 123.

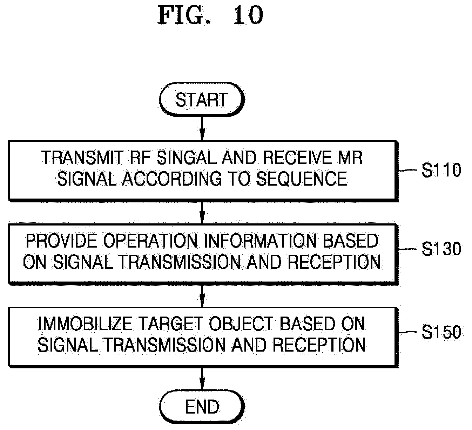

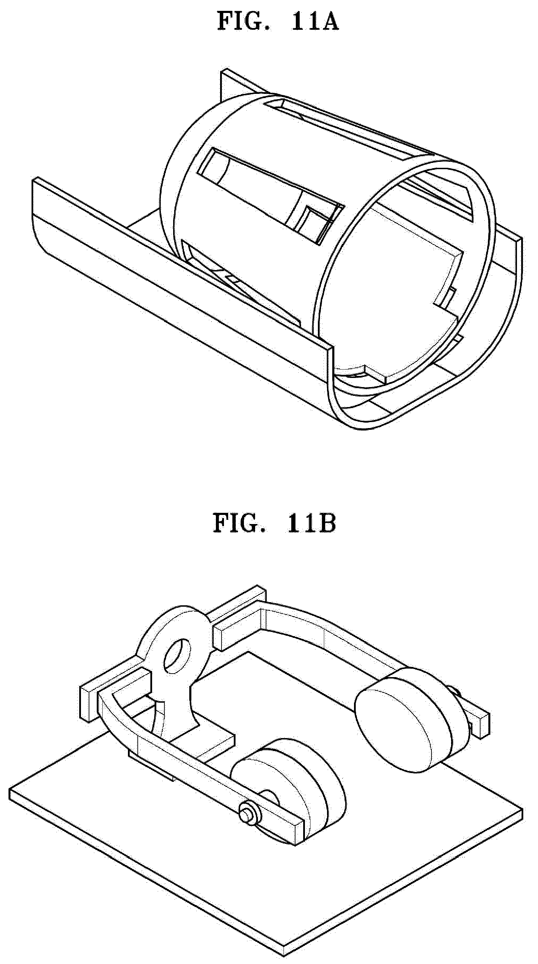

An operation of the immobilizer will now be described in more detail with reference to FIGS. 10, 11A, and 11B.

FIG. 10 is a flowchart of a method of controlling the MRI apparatus 100b according to another exemplary embodiment.

Referring to FIG. 10, in operation S110, the MRI apparatus 100 transmits an RF signal to and receives an MR signal from a target object according to a sequence. In operation S130, the MRI apparatus 100 provides operation information to the target object based on the signal transmission and reception.

In operation S150, the MRI apparatus 100 may prevent or immobilize, via the immobilizer 123, motion of the target object based on the signal transmission and reception. According to another exemplary embodiment, the MRI apparatus 100 may prevent motion of the target object, via the immobilizer 123, by skipping operation S130, i.e., without providing the operation information.

FIGS. 11A and 11B are perspective views of immobilizers in the MRI apparatus 100b according to exemplary embodiments. For example, the immobilizer 123 may be shaped as shown in FIG. 11A, so that it may be pulled down from a top side to a bottom side of a head to immobilize the head. As another example, the immobilizer 123 may be shaped as shown in FIG. 11B to immobilize the head securely from left and right sides thereof.

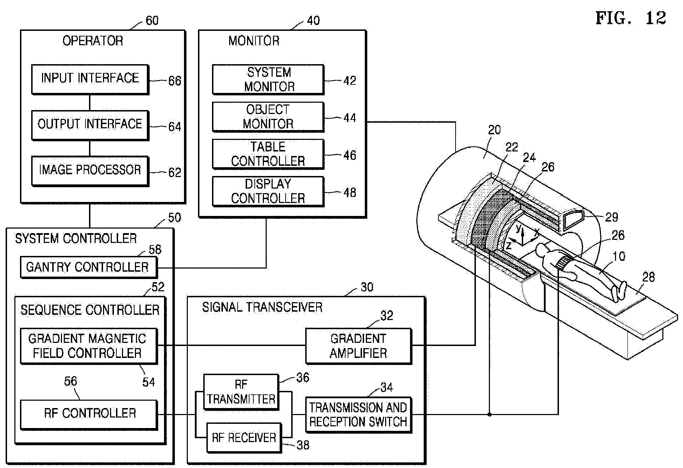

FIG. 12 is a block diagram of an MRI system. Referring to FIG. 1, the MRI system includes a gantry 20, a signal transceiver 30, a monitor 40, a system controller 50, and an operator 60.

The gantry 20 prevents external emission of electromagnetic waves generated by a main magnet 22, a gradient coil 24, and an RF coil 26. A magnetostatic field and a gradient magnetic field are formed in a bore in the gantry 20, and an RF signal is emitted toward an object 10.

The main magnet 22, the gradient coil 24, and the RF coil 26 may be arranged in a predetermined direction of the gantry 20. The predetermined direction may be a coaxial cylinder direction. The object 10 is disposed on a table 28 that is capable of being inserted into a cylinder along a horizontal axis of the cylinder.

The main magnet 22 generates a magnetostatic field or a static magnetic field for aligning magnetic dipole moments of atomic nuclei of the object 10 in a constant direction. A precise and accurate MR image of the object 10 may be obtained due to a magnetic field generated by the main magnet 22 being strong and uniform.

The gradient coil 24 includes X, Y, and Z coils for generating gradient magnetic fields in X-, Y-, and Z-axis directions crossing each other at right angles. The gradient coil 24 may provide location information of each region of the object 10 by differently inducing resonance frequencies according to the regions of the object 10.

The RF coil 26 may emit an RF signal toward a patient and receive an MR signal emitted from the patient. In detail, the RF coil 26 may transmit, toward atomic nuclei included in the patient and having precessional motion, an RF signal having the same frequency as that of the precessional motion, stop transmitting the RF signal, and then receive an MR signal emitted from the atomic nuclei included in the patient.

For example, to transit an atomic nucleus from a low energy state to a high energy state, the RF coil 26 may generate and apply an electromagnetic wave signal that is an RF signal corresponding to a type of the atomic nucleus, to the object 10. When the electromagnetic wave signal generated by the RF coil 26 is applied to the atomic nucleus, the atomic nucleus may transit from the low energy state to the high energy state. Then, when electromagnetic waves generated by the RF coil 26 disappear, the atomic nucleus to which the electromagnetic waves were applied transits from the high energy state to the low energy state, thereby emitting electromagnetic waves having a Lamor frequency. In other words, when the applying of the electromagnetic wave signal to the atomic nucleus is stopped, an energy level of the atomic nucleus is changed from a high energy level to a low energy level, and thus the atomic nucleus may emit electromagnetic waves having a Lamor frequency. The RF coil 26 may receive electromagnetic wave signals from atomic nuclei included in the object 10.

The RF coil 26 may be realized as one RF transmitting and receiving coil having both a function of generating electromagnetic waves each having an RF that corresponds to a type of an atomic nucleus and a function of receiving electromagnetic waves emitted from an atomic nucleus. Alternatively, the RF coil 26 may be realized as a transmission RF coil having a function of generating electromagnetic waves each having an RF that corresponds to a type of an atomic nucleus, and a reception RF coil having a function of receiving electromagnetic waves emitted from an atomic nucleus.

The RF coil 26 may be fixed to the gantry 20 or may be detachable. When the RF coil 26 is detachable, the RF coil 26 may be an RF coil for a part of the object, such as a head RF coil, a chest RF coil, a leg RF coil, a neck RF coil, a shoulder RF coil, a wrist RF coil, or an ankle RF coil.

The RF coil 26 may communicate with an external apparatus via wires and/or wirelessly, and may also perform dual tune communication according to a communication frequency band.

The RF coil 26 may be a birdcage coil, a surface coil, or a transverse electromagnetic (TEM) coil according to structures.

The RF coil 26 may be a transmission exclusive coil, a reception exclusive coil, or a transmission and reception coil according to methods of transmitting and receiving an RF signal.

The RF coil 26 may be an RF coil having various numbers of channels, such as 16 channels, 32 channels, 72 channels, and 144 channels.

The gantry 20 further includes a display 29 disposed outside the gantry 20, and may further include a display disposed inside the gantry 20. The gantry 20 may provide predetermined information to the user or the object 10 through the display 29 and the display respectively disposed outside and inside the gantry 20.

The signal transceiver 30 may control the gradient magnetic field formed inside the gantry 20, i.e., in the bore, according to a predetermined MR sequence, and control transmission and reception of an RF signal and an MR signal.

The signal transceiver 30 includes a gradient amplifier 32, a transmission and reception switch 34, an RF transmitter 36, and an RF receiver 38.

The gradient amplifier 32 drives the gradient coil 24 included in the gantry 20, and may supply a pulse signal for generating a gradient magnetic field to the gradient coil 24 under the control of a gradient magnetic field controller 54. By controlling the pulse signal supplied from the gradient amplifier 32 to the gradient coil 24, gradient magnetic fields in X-, Y-, and Z-axis directions may be synthesized.

The RF transmitter 36 and the RF receiver 38 may drive the RF coil 26. The RF transmitter 36 may supply an RF pulse in a Lamor frequency to the RF coil 26, and the RF receiver 38 may receive an MR signal received by the RF coil 26.

The transmission and reception switch 34 may adjust transmitting and receiving directions of the RF signal and the MR signal. For example, the transmission and reception switch 34 may emit the RF signal toward the object 10 through the RF coil 26 during a transmission mode, and receive the MR signal from the object 10 through the RF coil 26 during a reception mode. The transmission and reception switch 34 may be controlled by a control signal output by an RF controller 56.

The monitor 40 may monitor or control the gantry 20 or devices mounted on the gantry 20. The monitor 40 includes a system monitor 42, an object monitor 44, a table controller 46, and a display controller 48.

The system monitor 42 may monitor and control a state of the magnetostatic field, a state of the gradient magnetic field, a state of the RF signal, a state of the RF coil 26, a state of the table 28, a state of a device measuring body information of the object 10, a power supply state, a state of a thermal exchanger, and a state of a compressor.

The object monitor 44 monitors a state of the object 10. In detail, the object monitor 44 may include a camera for observing a movement or position of the object 10, a respiration measurer for measuring the respiration of the object 10, an electrocardiogram (ECG) measurer for measuring the electrical activity of the object 10, or a temperature measurer for measuring a temperature of the object 10.

The table controller 46 controls a movement of the table 28 where the object 10 is positioned. The table controller 46 may control the movement of the table 28 according to a sequence control of a sequence controller 50. For example, during moving imaging of the object 10, the table controller 46 may continuously or discontinuously move the table 28 according to the sequence control of the sequence controller 50, and thus the object 10 may be photographed in a field of view (FOV) larger than that of the gantry 20.

In an exemplary embodiment, the table controller 46 may operate according to control by the gantry controller 58. The table controller 46 may control the immobilizer 123 according to a sequence of operations of the controller 150. Thus, a high quality image may be obtained by directly preventing motion of the target object.

The display controller 48 controls the display 29 disposed outside the gantry 20 and the display disposed inside the gantry 20. In detail, the display controller 48 may control the display 29 and the display to be on or off, and may control a screen image to be output on the display 29 and the display. Also, when a speaker is located inside or outside the gantry 20, the display controller 48 may control the speaker to be on or off, or may control sound to be output via the speaker.

In an exemplary embodiment, the display controller 48 may operate according to control by the gantry controller 58. The display controller 48 may display 121 according to a sequence of operations of the controller 150. Thus, the target object may view an image and make a conscious effort to not move, and thus, a high quality image may be obtained.

The system controller 50 includes a sequence controller 52 for controlling a sequence of signals formed in the gantry 20, and a gantry controller 58 for controlling the gantry 20 and the devices mounted on the gantry 20.

The sequence controller 52 includes the gradient magnetic field controller 54 for controlling the gradient amplifier 32, and the RF controller 56 for controlling the RF transmitter 36, the RF receiver 38, and the transmission and reception switch 34. The sequence controller 52 may control the gradient amplifier 32, the RF transmitter 36, the RF receiver 38, and the transmission and reception switch 34 according to a pulse sequence received from the operator 60. The pulse sequence includes all information used to control the gradient amplifier 32, the RF transmitter 36, the RF receiver 38, and the transmission and reception switch 34. For example, the pulse sequence may include information about a strength, an application time, and application timing of a pulse signal applied to the gradient coil 24.

The operator 60 may request the system controller 50 to transmit pulse sequence information while controlling an overall operation of the MRI system.

The operator 60 includes an image processor 62 for receiving and processing the MR signal received by the RF receiver 38, an output interface 64, and an input interface 66.

The image processor 62 may process the MR signal received from the RF receiver 38 to generate MR image data of the object 10.

The image processor 62 receives the MR signal received by the RF receiver 38 and performs any one of various signal processes, such as amplification, frequency transformation, phase detection, low frequency amplification, and filtering, on the received MR signal.

The image processor 62 may arrange digital data in a k space (for example, also referred to as a Fourier space or a frequency space) of a memory, and rearrange the digital data into image data via 2D or 3D Fourier transformation.

The image processor 62 may perform a composition process or difference calculation process on the image data. The composition process may include an addition process on a pixel or a maximum intensity projection (MIP) process. The image processor 62 may store not only the rearranged image data but also image data on which a composition process or a difference calculation process is performed, in a memory or an external server.

The image processor 62 may perform any of the signal processes on the MR signal in parallel. For example, the image processor 62 may perform a signal process on a plurality of MR signals received by a multi-channel RF coil in parallel to rearrange the plurality of MR signals into image data.

The output interface 64 may output image data generated or rearranged by the image processor 62 to the user. The output interface 64 may also output information used for the user to manipulate the MRI system, such as a user interface (UI), user information, or object information. The output interface 64 may be a speaker, a printer, a cathode-ray tube (CRT) display, a liquid crystal display (LCD), a plasma display panel (PDP), an organic light-emitting device (OLED) display, a field emission display (FED), a light-emitting diode (LED) display, a vacuum fluorescent display (VFD), a digital light processing (DLP) display, a flat panel display (FPD), a 3D display, a transparent display, or any one of other various output devices.

The user may input object information, parameter information, a scan condition, a pulse sequence, or information about image composition or difference calculation by using the input interface 66. The input interface 66 may be a keyboard, a mouse, a track ball, a voice recognizer, a gesture recognizer, a touch screen, or any one of other various input devices.

The signal transceiver 30, the monitor 40, the system controller 50, and the operator 60 are separate components in FIG. 12, but respective functions of the signal transceiver 30, the monitor 40, the system controller 50, and the operator 60 may be performed by another component. For example, the image processor 62 converts the MR signal received from the RF receiver 38 into a digital signal in FIG. 12, but alternatively, the conversion of the MR signal into the digital signal may be performed by the RF receiver 38 or the RF coil 26.

The gantry 20, the RF coil 26, the signal transceiver 30, the monitor 40, the system controller 50, and the operator 60 may be connected to each other by wire or wirelessly, and when they are connected wirelessly, the MRI system may further include an apparatus for synchronizing clock signals therebetween. Communication between the gantry 20, the RF coil 26, the signal transceiver 30, the monitor 40, the system controller 50, and the operator 60 may be performed by using a high-speed digital interface, such as low voltage differential signaling (LVDS), asynchronous serial communication, such as a universal asynchronous receiver transmitter (UART), a low-delay network protocol, such as error synchronous serial communication or a controller area network (CAN), optical communication, or any of other various communication methods.

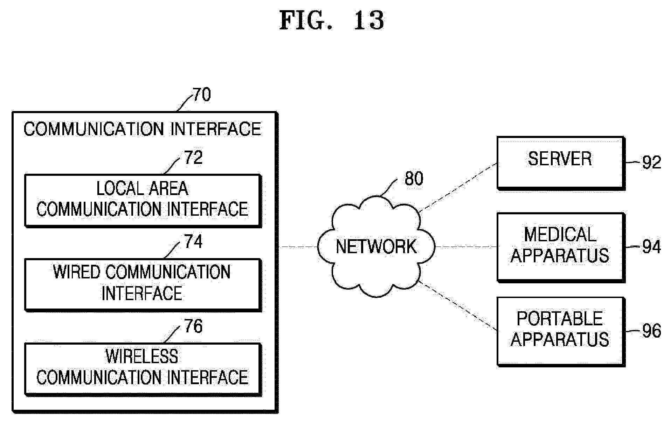

FIG. 13 is a block diagram of a communication interface 70 according to an exemplary embodiment. Referring to FIG. 13, the communication interface 70 may be connected to at least one selected from the gantry 20, the signal transceiver 30, the monitor 40, the system controller 50, and the operator 60 of FIG. 12.

The communication interface 70 may transmit and receive data to and from a hospital server or another medical apparatus in a hospital, which is connected through a picture archiving and communication system (PACS), and perform data communication according to the digital imaging and communications in medicine (DICOM) standard.

As shown in FIG. 13, the communication interface 70 may be connected to a network 80 by wire or wirelessly to communicate with a server 92, a medical apparatus 94, or a portable device 96.

In detail, the communication interface 70 may transmit and receive data related to the diagnosis of an object through the network 80, and may also transmit and receive a medical image captured by the medical apparatus 94, such as a CT apparatus, an MRI apparatus, or an X-ray apparatus. In addition, the communication interface 70 may receive a diagnosis history or a treatment schedule of the object from the server 92 and use the same to diagnose the object. The communication interface 70 may perform data communication not only with the server 92 or the medical apparatus 94 in a hospital, but also with the portable device 96, such as a mobile phone, a personal digital assistant (PDA), or a laptop of a doctor or patient.

Also, the communication interface 70 may transmit information about a malfunction of the MRI system or about a medical image quality to a user through the network 80, and receive a feedback regarding the information from the user.

The communication interface 70 may include at least one component enabling communication with an external apparatus.

For example, the communication interface 70 includes a local area communication interface 72, a wired communication interface 74, and a wireless communication interface 76. The local area communication interface 72 refers to an interface for performing local area communication with an apparatus within a predetermined distance. Examples of local area communication technology according to an exemplary embodiment include, but are not limited to, a wireless local area network (LAN), Wi-Fi, Bluetooth, ZigBee, Wi-Fi direct (WFD), ultra wideband (UWB), infrared data association (IrDA), Bluetooth low energy (BLE), and near field communication (NFC).

The wired communication interface 74 refers to an interface for performing communication by using an electric signal or an optical signal. Examples of wired communication technology according to an exemplary embodiment include wired communication techniques using a pair cable, a coaxial cable, and an optical fiber cable, and other wired communication techniques.

The wireless communication interface 76 transmits and receives a wireless signal to and from at least one selected from a base station, an external apparatus, and a server in a mobile communication network. The wireless signal may be a voice call signal, a video call signal, or data in any one of various formats according to transmission and reception of a text/multimedia message.

In addition, the exemplary embodiments may also be implemented through computer-readable code and/or instructions on a medium, e.g., a computer-readable medium, to control at least one processing element to implement any above-described embodiments. The medium may correspond to any medium or media that may serve as a storage and/or perform transmission of the computer-readable code.

The computer-readable code may be recorded and/or transferred on a medium in a variety of ways, and examples of the medium include recording media, such as magnetic storage media (e.g., ROM, floppy disks, hard disks, etc.) and optical recording media (e.g., compact disc read only memories (CD-ROMs) or digital versatile discs (DVDs)), and transmission media such as Internet transmission media. Thus, the medium may have a structure suitable for storing or carrying a signal or information, such as a device carrying a bitstream according to one or more exemplary embodiments. The medium may also be on a distributed network, so that the computer-readable code is stored and/or transferred on the medium and executed in a distributed fashion. Furthermore, the processing element may include a processor or a computer processor, and the processing element may be distributed and/or included in a single device.

The foregoing exemplary embodiments are examples and are not to be construed as limiting. The present teaching can be readily applied to other types of apparatuses. Also, the description of the exemplary embodiments is intended to be illustrative, and not to limit the scope of the claims, and many alternatives, modifications, and variations will be apparent to those skilled in the art

## Claims

1. A magnetic resonance imaging (MRI) apparatus comprising:
a signal transceiver configured to transmit a radio frequency (RF) signal to an object, and receive a magnetic resonance (MR) signal from the object; and
a controller configured to:
control the signal transceiver based on sequence information that is used according to an imaging protocol;
determine a degree at which the object is able to move based on the sequence information, to generate operation information; and
control a device connected to the MRI apparatus to provide the operation information.

2. The MRI apparatus of claim 1, wherein the controller is further configured to determine information of whether the object is able to move based on a time interval.

3. The MRI apparatus of claim 1, wherein the device comprises a display configured to display the operation information to the object as an image.

4. The MRI apparatus of claim 3, wherein the controller is further configured to control the display to display the operation information to the object during a time interval when the object is able to move.

5. The MRI apparatus of claim 3, wherein the controller is further configured to control the display to:
display an image for inducing a concentration of the object while the signal transceiver is receiving MR signals of a low frequency region in a k-space; and
display an image for helping the object relax while the signal transceiver is receiving MR signals of a high frequency region in the k-space.

6. The MRI apparatus of claim 3, wherein the controller is further configured to control the display to display an image showing a level of progress of an examination of the object based on the operation information.

7. The MRI apparatus of claim 6, wherein the image comprises at least one among a progress bar and a text message.

8. The MRI apparatus of claim 6, wherein the image distinguishes a first time interval when the object is to remain stationary from a second time interval when the object is able to move.

9. A method of controlling a magnetic resonance imaging (MRI) apparatus, the method comprising:
transmitting a radio frequency (RF) signal to an object, and receiving a magnetic resonance (MR) signal from the object, based on sequence information that is used according to an imaging protocol;
determining a degree at which the object is able to move based on the sequence information, to generate operation information; and
providing the operation information.

10. The method of claim 9, wherein the determining comprises determining information of whether the object is able to move based on a time interval.

11. The method of claim 9, wherein the providing comprises displaying the operation information to the object as an image.

12. The method of claim 11, wherein the providing comprises displaying the operation information to the object during a time interval when the object is able to move.

13. The method of claim 11, wherein the providing comprises:
displaying an image for inducing a concentration of the object while the signal transceiver is receiving MR signals of a low frequency region in a k-space; and
displaying an image for helping the object relax while the signal transceiver is receiving MR signals of a high frequency region in the k-space.

14. The method of claim 11, wherein the providing comprises displaying an image showing a level of progress of an examination of the object based on the operation information.

15. The method of claim 14, wherein the image comprises at least one among a progress bar and a text message.
